# EUROPEAN PATENT SPECIFICATION

(11) **EP 2 382 341 B1**
(45) Date of publication and mention of the grant of the patent: **24.05.2017**
(21) Application number: 10701617.2
(22) Date of filing: 15.01.2010
(51) Int. Cl.: C25F 3/16, A61L 31/00

(54) **METHOD, APPARATUS AND SOLUTION FOR ELECTROPOLISHING METALLIC STENTS**
VERFAHREN, VORRICHTUNG UND LÖSUNG ZUM ELEKTROPOLIEREN VON METALLISCHEN STENTS
PROCÉDÉ, APPAREIL ET SOLUTION POUR LE POLISSAGE ÉLECTROLYTIQUE D'ENDOPROTHÈSES MÉTALLIQUES

(30) Priority: 16.01.2009 US 145453 P
(43) Date of publication of application: 02.11.2011
(73) Proprietor: Abbott Laboratories Vascular Enterprises Limited, Dublin 2 (IE)
(72) Inventor: WULF, Elena, 72393 Burladingen (DE)
(74) Representative: Peters, Hajo
(86) International application number: PCT/EP2010/000216
(87) International publication number: WO 2010/081723

(56) References cited:
- US-A- 3 190 822
- US-A1- 2005 209 117
- US-A1- 2007 209 947
- US-B1- 6 679 980
- US-B1- 6 916 409

## Description

### BACKGROUND OF THE DISCLOSURE

The present disclosure relates generally to providing an electrolytic solution, as well as an apparatus and a method with such an electrolytic solution for electropolishing products made from metals, and in particular,

### BACKGROUND OF THE DISCLOSURE

The present disclosure relates generally to providing an apparatus and method for electropolishing products made from metals, and in particular, electropolishing metallic medical devices such as stents, made of stainless steel, titanium, tungsten, nickel-titanium, tantalum, cobalt-chromium-tungsten, etc. While the apparatus and method are described herein as being applicable mainly to medical stents, in particular intravascular stents, the disclosure is not limited to such medical products. For example, the methods may be applied to electropolish metallic automotive or aerospace components.

Stents are generally tube-shaped devices placed within a blood vessel or other body lumen to maintain the patency of the lumen and, in some cases, to reduce the development of restenosis. The stents may be formed in a variety of configurations which are typically expandable since they are delivered in a compressed form to the desired site. Such a configuration may be a helically wound wire, wire mesh, weaved wire, serpentine stent, chain of rings, other configurations, or combinations thereof. The walls of stents are typically perforated in a framework design of wire-like connected elements or struts or in a weave design of cross-threaded wire. Some stents are made of more than one material. The stent may be, for example, a sandwich of metals having outer layers of a biocompatible material, such as stainless steel, with an inner layer providing the radiopacity to the stent for tracking by imaging devices during placement. A stent made of such material may be, for example, a thin layer of titanium between layers of stainless steel. In forming such stents from metal, a roughened outer surface of the stent may result from the manufacturing process. It may be desirable for the surface of the stent to be smooth so that it may be more readily inserted and traversed with reduced friction through the blood vessels or other body lumens toward the site of implantation. A rough outer surface may not only increase frictional obstruction, but may also damage the lining of the vessel wall during insertion. Furthermore, smooth surfaces may reduce thrombus formation and/or corrosion.

Since processing to form metallic stents often results in a product initially having burrs, sharp ends, debris, slag material from melting the metal during processing, other features, or combinations thereof, as a first order treatment of the product, descaling of the surface may be descaled in preparation for, for example, further surface treatment such as electropolishing.

An apparatus and method is provided for electropolishing stents after they have been descaled, for example, by the method disclosed in US 2007/0209947 A1.

Descaling may include, for example, dipping the stent into a strongly acidic solution and/or ultrasonically cleaning the stent.

Electropolishing is an electrochemical process by which some of the surface metal may be electrolytically dissolved. In general, the metal stent serves as an anode and is connected to a power supply while immersed in an electrolytic solution having a metal cathode connected to the negative terminal of the power supply. Current therefore flows from the stent, as the anode, causing it to become polarized. The rate at which the metal ions on the stent are dissolved may be controlled by the applied current and/or voltage. The positioning of the cathode relative to the stent may provide an even distribution of current to the stent. According to the theory of electropolishing, the current density is typically highest at high points protruding from a surface and is typically lowest at the surface low points. Thus, the higher current density at the raised points may cause the metal to dissolve faster at these points, which may level the surface. Electropolishing therefore may smooth the surface, even to the point where it is shiny and reflective.

US 2007/0209947 A1 discloses an apparatus for simultaneously electropolishing a plurality of metallic stents comprising a plurality of elongated members having a longitudinal axis, the members comprising an electrically conductive adaptor capable of being removably affixed to and in electrical contact with a metallic stent; an electrolytic solution contained in a polishing container; a cathode configured to be located in close proximity to the elongated members when said elongated members and cathode are immersed in said electrolytic solution; a cathode current conducting member attached to said cathode; an anode current conducting member wherein the elongated members are conductively connected electrically with said anode current conducting member. Further, US 2007/0209947 A1 discloses a method for simultaneously electropolishing metallic stents comprising the steps of affixing a stent on electrically conductive adaptors of an apparatus which comprises an elongated member having a longitudinal axis, the members comprising an electrically conductive adaptor capable of being removably affixed to and in electrical contact with a metallic stent; an electrolytic solution; a continuous cathode configured to be located in close proximity to the elongated members when the member and the cathode are immersed in said electrolytic solution; a cathode current conducting member attached to said cathode; an anode current conducting member wherein each of said elongated members is conductively connected electrically with said anode current conducting member; immersing the stents in said electrolytic solution; supplying a voltage difference between said cathode current conducting member and said anode current conducting member; and removing the stents from said solution and rinsing with alcohol.

US 2007/0209947 A1 does not disclose that the electrolytic solution comprises polyethylene glycol, ethanol, and dimethylsulfate, and a continuous spiral cathode.

US 6,679,980 B1 discloses an apparatus for electropolishing a stent. The cathode used in this apparatus has a spiral shape.

The present disclosure provides an electrolytic solution, and an apparatus and process for electropolishing a plurality of metallic devices using such an electrolytic solution.

### BRIEF SUMMARY

The invention provides an electrolytic solution comprising polyethylene glycol, ethanol and dimethylsulfate. Further, the invention provides an apparatus according to claim 1 for simultaneously electropolishing a plurality of metallic stents, and a method according to claim 5 for simultaneously electropolishing metallic stents.

In one embodiment, the container is made from non-metallic material. The container may be made from glass, ceramics, plastic, or other materials.

In another embodiment, the apparatus includes a second continuous cathode configured to be located in close proximity to each of the elongated members when the elongated members and cathode are immersed in the electrolytic solution and a second cathode current conducting member attached to the second cathode.

In a further embodiment, the two cathodes are both in the shape of a spiral and are disposed substantially concentrically in the solution.

In one embodiment, the electrolytic solution may include from about 0.1 to about 5 weight percent polyethylene glycol, from about 0.5 to about 2 weight percent polyethylene glycol, or about 1 weight percent polyethylene glycol. In a further embodiment, the polyethylene glycol is PEG 1000.

In one embodiment, the electrolytic solution may include from about 0.1 to about 5 weight percent polyethylene glycol, from about 0.5 to about 2 weight percent polyethylene glycol, or about 1 weight percent polyethylene glycol, where the polyethylene glycol is PEG 1000; from about 40 to about 70 weight percent dimethylsulfate, from about 50 to about 60 weight percent, from about 54 to about 58 weight percent, from about 55 to about 57 weight percent; and a balance of Ethanol.

In another embodiment, in act c) of claim 5, the voltage is supplied for a period in the range of about 20 to about 60 seconds while the stents are immersed in the electrolytic solution.

In one embodiment, the passivation solution includes nitric acid.

These and other objects and features of the present disclosure will become more fully apparent from the following description and appended claims, or may be learned by the practice of the disclosure as set forth hereinafter.

### BRIEF DESCRIPTION OF THE DRAWINGS

To further clarify the above and other advantages and features of the present disclosure, a more particular description of the disclosure will be rendered by reference to specific embodiments thereof which are illustrated in the appended drawings. It is appreciated that these drawings depict only illustrated embodiments of the disclosure and are therefore not to be considered limiting of its scope. The disclosure will be described and explained with additional specificity and detail through the use of the accompanying drawings in which:

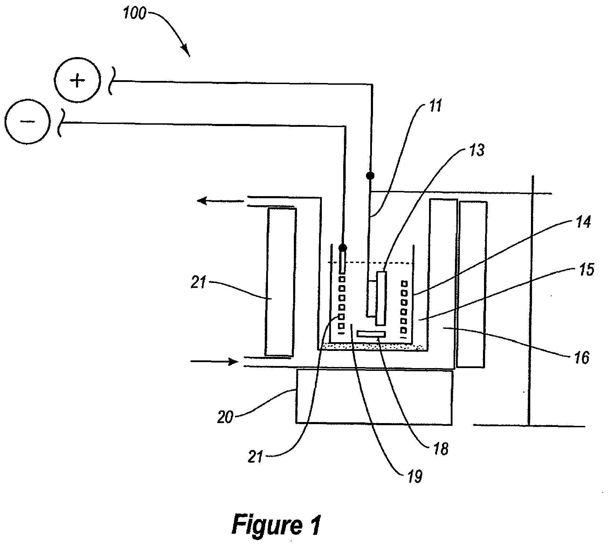

Figure 1 illustrates a view of an apparatus according to the disclosure showing the stents immersed in electrolytic solution and in close proximity to the cathode.

### DETAILED DESCRIPTION

The present disclosure is directed to an apparatus and method for electropolishing a plurality of metallic devices, in particular, metallic stents. The present disclosure may provide the advantage of simultaneously electropolishing a plurality of devices. By providing a spiral cathode in an electropolishing container made from a material with low thermal conduction, the electrolyte solutions may remain stable for an extended time.

In yet another embodiment, the apparatus includes two concentric spiral cathodes with the stents, as anodes, placed therebetween, thereby providing additional cathode surface area. The pitch of the spiral can be varied in order to balance good agitation of the electrolytic solution in the polishing container to ensure continuous electrolyte concentration and composition and provision of a high surface area of the cathode.

A novel and improved electrolytic solution for improved electropolishing of metallic medical devices, in particular stents is also described.

Referring to FIG. 1, there is shown an embodiment of an apparatus 100 according to the disclosure. The apparatus may include at least one elongated member 11 in a downward orientation along a longitudinal axis. Each of the members 11 may accommodate an electrically conductive adapter which may be capable of being removably affixed to and/or in electrical contact with a metallic stent 13.

With the at least one elongated member 11 immersed in electropolishing solution 19 contained in a container 14, a tubular continuous spiral cathode 21 may be in close proximity with the at least one elongated members 11. Each member 11 may be substantially equidistant from the facing surface of the cathode 21. This may provide a consistent field to each of the stents. Cathode 21 may be attached to a cathode collecting member. The at least one stent, as anodes, attached to elongated member(s) 11 may be all in electrical connection in series and/or in parallel with anode current conducting member. Cathode conducting member and/or anode conducting member may be connected to an electromotive force (EMF)-providing DC source, such as a battery, from which current and/or voltage may be controlled by an appropriate controller. The entire system may be placed into a polishing container 14 made from material with a low thermal conduction. Suitable materials may include glass, ceramics, plastic, or other materials. The polishing container 14 may contain the electropolishing solution 19 and/or may be placed in a double walled reaction container 16. The polishing container 14 may be cooled with a cooling solution 15 like e.g. Ethanol. Using a polishing container 14 made from material with a low thermal conduction water may reduce condensation at the wall portions of the polishing container exposed. A dilution of the polishing solution by condensing water out of the air may be reduced, potentially resulting in an electropolishing solution 19 that may be stable and/or reliable and/or may provide constant polishing results. To facilitate agitation of the electropolishing solution during the electropolishing process, a stirrer 18, such as a magnetic stirrer may be placed into the polishing container 14.

Typical coronary and/or endovascular stents may vary in a range from about 7 to up to about 200 millimeters in length with a diameter in a range of about 1 to about 12 millimeters. However, stents of larger or smaller size may be suitably accommodated.

In order to accomplish the electropolishing process, the stents, which may all be identical in length, diameter, design, or combinations thereof, may be placed on one or more of the adaptors. The mounted stents may be immersed into the electropolishing solution. The temperature of the electropolishing solution may be between about -20°C and about 0°C, between about -20°C and about -10°C, or about-15°C. A voltage is supplied to the stents, as anodes, and the cathode to electropolish the stents to the desired smoothness. Useful voltage may be in the range of about 7 to about 40 volts, between about 10 and about 30 volts, or between about 10 and about 20 volts. Voltage may be applied in about 20 to about 60 second intervals, in some embodiments in about 30 second intervals. However, voltages outside of these ranges may also be useful, depending upon the number of stents, electrolyte, and/or other design and/or process parameters.

It may be desirable for the electropolishing process to be performed in stages. After one immersion in the electropolishing solution, topically lasting from about 20 to about 60 seconds, the stents may be removed from the solution and washed, typically with alcohol, water, nitric acid, or combinations thereof. Then, the electropolishing may be repeated several times with each step followed by a rinse of the stents. Typically a suitable polishing process will include about three to about five iterations of the electropolishing step. But more or fewer iterations may be suitable, depending upon the stents, electrolyte, voltage, other process variations, or combinations thereof. Once the desired electropolishing is completed, the stents may be removed from the electropolishing solution and from the electropolishing apparatus, rinsed, and contacted with a passivation solution to remove residual electropolishing solution. The stents are typically again rinsed and placed in a bath to which ultrasound energy may be applied to complete the rinsing. A final rinse step may involve exposure for about 10 minutes in an ultrasound bath at approximately room temperature.

An embodiment of a solution may include polyethylene glycol, ethanol, and dimethylsulfate.

In a further embodiment, the electrolytic solution may include from about 0.1 to about 5 weight percent polyethylene glycol, from about 0.5 to about 2 weight percent polyethylene glycol, or about 1 weight percent polyethylene glycol. In a still further embodiment, the polyethylene glycol is PEG 1000.

In a further embodiment, the electrolytic solution includes from about 40 to about 70 weight percent dimethylsulfate, from about 50 to about 60 weight percent, from about 54 to about 58 weight percent, or from about 56 to about 57 weight percent; and a balance of ethanol.

In a further embodiment, the electrolytic solution includes from about 0.1 to about 5 weight percent polyethylene glycol, from about 0.5 to 2 weight percent polyethylene glycol, or about 1 weight percent polyethylene glycol, wherein polyethylene glycol is PEG 1000; from about 40 to about 70 weight percent dimethylsulfate, from about 50 to about 60 weight percent, from about 54 to about 58 weight percent, from about 55 to about 57 weight percent, or about 56 to about 57 weight percent; and a balance of ethanol.

The following example is presented for the purpose of illustration and is not intended to limit the disclosure in any way.

### EXAMPLE

Four dry identical stents may be removably affixed to the adapters of four elongated members. While agitating the electropolishing solution (polyethylene glycol (PEG 1000) : dimethylsulfate : ethanol in a weight ratio of about 1 : 59 : 44 or in other words about 1 weight percent polyethylene glycol (PEG 1000), 57 weight percent dimethylsulfate, 42 weight percent ethanol) the stents are lowered on the apparatus into the electropolishing solution. The positive lead from the electrical source is attached to the apparatus and the magnetic stirrer in the electropolishing container is turned on to facilitate agitation of the electropolishing solution. When the cycle time has elapsed (depending on the size and type of stent), the stents are removed from the electropolishing solution and submerged in a container of ethanol. Each stent is moved while submerged. The stents are then re-immersed in the electropolishing solution for another polishing cycle. The polishing cycle is repeated for four polishing cycles. The stents are removed from the adapters and placed into a purified water rinse for about 30 seconds. The stents are then removed and placed in nitric acid passivation rinse bath for 30 minutes. The stents are removed from the bath and placed in a purified water ultrasonic bath for about 10 minutes. The stents are then removed from the bath, rinsed with alcohol and are dried with compressed air.

The present disclosure may be embodied in other specific forms without departing from its essential characteristics. The described embodiments are to be considered in all respects only as illustrative and not restrictive. The scope of the disclosure is, therefore, indicated by the appended claims rather than by the foregoing description.

## Claims

1. An apparatus (100) for simultaneously electropolishing a plurality of metallic stents (13) comprising:
a plurality of elongated members (11) having a longitudinal axis, each of said members (11) comprising an electrically conductive adaptor capable of being removably affixed to and in electrical contact with a metallic stent (13);
an electrolytic solution (19) comprising polyethylene glycol, ethanol, and dimethylsulfate contained in a polishing container (14);
a continuous spiral cathode (21) configured to be located in close proximity to each of said elongated members (11) when said elongated members (11) and cathode (21) are immersed in said electrolytic solution (19);
a cathode current conducting member attached to said cathode (21);
an anode current conducting member wherein each of said elongated members (11) is conductively connected electrically with said anode current conducting member.

2. The apparatus (100) according to claim 1, wherein the polishing container (14) is made from material with low thermal conductivity.

3. The apparatus (100) according to any of claims 1 or 2, further comprising a second continuous spiral cathode (21) configured to be located in close proximity to each of said elongated members (11) when said elongated members (11) and cathode (21) are immersed in said electrolytic solution (19); and a second spiral cathode current conducting member attached to said second cathode.

4. The apparatus (100) according to claim 3, wherein said spiral cathodes (21) are tubular in shape and disposed substantially concentrically in said solution (19).

5. A method of simultaneously electropolishing metallic stents (13) comprising:
a) affixing a stent (13) on each of one or more of electrically conductive adaptors of an apparatus (100), said apparatus (100) comprising:
at least one elongated member (11) having a longitudinal axis, each of said at least one member (11) comprising an electrically conductive adaptor capable of being removably affixed to and in electrical contact with a metallic stent (13);
an electrolytic solution (19) comprising polyethylene glycol, ethanol, and dimethylsulfate;
a continuous spiral cathode (21) configured to be located in close proximity to each of said at least one elongated member (11) when said a least one elongated member (11) and cathode (21) are immersed in said electrolytic solution (19);
a cathode current conducting member attached to said cathode (21);
an anode current conducting member wherein each of said elongated members (11) is conductively connected electrically with said anode current conducting member;
b) immersing said at least one stent (13) in said electrolytic solution (19);
c) supplying a voltage difference between said cathode current conducting member and said anode current conducting member;
d) removing said at least one stent (13) from said solution (19) and rinsing with alcohol; and
e) optionally, repeating acts b), c), and d).

6. The method according to claim 5, further comprising the steps of
f) removing said at least one stent (13) from said apparatus (100);
g) rinsing said at least one stent (13);
h) immersing said at least one stent (13) in a passivation solution;
i) removing said at least one stent (13) from said passivation solution and rinsing said at least one stent (13); and
j) placing said at least one stent (13) in a liquid and applying ultrasound energy to said liquid.

7. The method according to claim 5, wherein said acts b), c), and d) are repeated three to five times.

8. The method according to claim 6, wherein said passivation solution comprises nitric acid.

9. The method according to claim 5, wherein said ultrasound energy is applied to said liquid at room temperature.

10. An electrolytic solution (19) comprising polyethylene glycol, ethanol and dimethylsulfate.

11. The electrolytic solution (19) according to claim 10, wherein the polyethylene glycol is PEG 1000.

12. The electrolytic solution (19) according to claims 10 or 11, wherein the electrolytic solution (19) comprises 0.1 to 5 weight percent polyethylene glycol.

13. The electrolytic solution (19) according to any of claims 10, 11, or 12, wherein the electrolytic solution (19) comprises 40 to 70 weight percent dimethylsulfate, and a balance of ethanol.

## Patentansprüche

1. Vorrichtung (100) zum gleichzeitigen Elektropolieren von mehreren metallischen Stents (13), aufweisend:
mehrere längliche Elemente (11) mit einer Längsachse, wobei jedes der Elemente (11) einen elektrisch leitenden Adapter aufweist, der sich abnehmbar an einem metallischen Stent (13) und in elektrischem Kontakt mit diesem befestigten lässt;
eine elektrolytische Lösung (19), die Polyethylenglykol, Ethanol und Dimethylsulfat aufweist, die in einem Polierbehälter (14) aufgenommen sind;
eine fortlaufende spiralförmige Kathode (21), die dafür ausgelegt ist, sich in unmittelbarer Nähe von jedem der länglichen Elemente (11) zu befinden, wenn die länglichen Elemente (11) und die Kathode (21) in der elektrolytischen Lösung (19) eingetaucht sind;
ein kathodenstromführendes Element, das an der Kathode (21) angebracht ist;
ein anodenstromführendes Element, wobei jedes der länglichen Elemente (11) elektrisch leitend mit dem anodenstromführenden Element verbunden ist.

2. Vorrichtung (100) nach Anspruch 1, wobei der Polierbehälter (14) aus einem Material geringer Wärmeleitfähigkeit hergestellt ist.

3. Vorrichtung (100) nach einem beliebigen der Ansprüche 1 oder 2, ferner aufweisend: eine zweite fortlaufende spiralförmige Kathode (21), die dafür ausgelegt ist, sich in unmittelbarer Nähe von jedem der länglichen Elemente (11) zu befinden, wenn die länglichen Elemente (11) und die Kathode (21) in der elektrolytischen Lösung (19) eingetaucht sind; und ein zweites spiralförmiges kathodenstromführendes Element, das an der zweiten Kathode angebracht ist.

4. Vorrichtung (100) nach Anspruch 3, wobei die spiralförmigen Kathoden (21) rohrförmig gestaltet sind und in der Lösung (19) im Wesentlichen konzentrisch angeordnet sind.

5. Verfahren zum gleichzeitigen Elektropolieren von metallischen Stents (13), mit folgenden Schritten:
a) Befestigen eines Stents (13) an jedem von dem einen oder den mehreren elektrisch leitenden Adaptern einer Vorrichtung (100), wobei die Vorrichtung (100) aufweist:
wenigstens ein längliches Element (11) mit einer Längsachse, wobei jedes von dem wenigstens einen Element (11) einen elektrisch leitenden Adapter aufweist, der sich abnehmbar an einem metallischen Stent (13) und in elektrischem Kontakt mit diesem befestigten lässt;
eine elektrolytische Lösung (19), die Polyethylenglykol, Ethanol und Dimethylsulfat enthält;
eine fortlaufende spiralförmige Kathode (21), die dafür ausgelegt ist, sich in unmittelbarer Nähe von jedem der wenigstens einen länglichen Elemente (11) zu befinden, wenn das wenigstens eine längliche Element (11) und die Kathode (21) in der elektrolytischen Lösung (19) eingetaucht sind;
ein kathodenstromführendes Element, das an der Kathode (21) angebracht ist;
ein anodenstromführendes Element, wobei jedes der länglichen Elemente (11) elektrisch leitend mit dem anodenstromführenden Element verbunden ist;
b) Eintauchen des wenigstens einen Stents (13) in die elektrolytische Lösung (19);
c) Anlegen einer Spannungsdifferenz zwischen dem kathodenstromführenden Element und dem anodenstromführenden Element;
d) Entfernen des wenigstens einen Stents (13) aus der Lösung (19) und Spülen mit Alkohol; und
e) optional, Wiederholen der Schritte b), c) und d).

6. Verfahren nach Anspruch 5, wobei zu dem Verfahren ferner die Schritte gehören:
f) Entfernen des wenigstens einen Stents (13) aus der Vorrichtung (100);
g) Spülen des wenigstens einen Stents (13);
h) Eintauchen des wenigstens einen Stents (13) in eine Passivierungslösung;
i) Entfernen des wenigstens einen Stents (13) aus der Passivierungslösung und Spülen des wenigstens einen Stents (13); und
j) Platzieren des wenigstens einen Stents (13) in einer Flüssigkeit und Übertragen von Ultraschallenergie auf die Flüssigkeit.

7. Verfahren nach Anspruch 5, wobei die Schritte b), c) und d) drei- bis fünfmal wiederholt werden.

8. Verfahren nach Anspruch 6, wobei die Passivierungslösung Salpetersäure enthält.

9. Verfahren nach Anspruch 5, wobei die Ultraschallenergie auf die Flüssigkeit bei Raumtemperatur übertragen wird.

10. Elektrolytische Lösung (19), die Polyethylenglykol, Ethanol und Dimethylsulfat enthält.

11. Elektrolytische Lösung (19) nach Anspruch 10, wobei das Polyethylenglykol PEG 1000 ist.

12. Elektrolytische Lösung (19) nach Anspruch 10 oder 11, wobei die elektrolytische Lösung (19) 0,1 bis 5 Gewichtsprozent Polyethylenglykol enthält.

13. Elektrolytische Lösung (19) nach einem beliebigen der Ansprüche 10, 11 oder 12, wobei die elektrolytische Lösung (19) 40 bis 70 Gewichtsprozent Dimethylsulfat enthält und der Rest Ethanol ist.

## Revendications

1. Appareil (100) de polissage électrolytique simultané d'une pluralité de stents métalliques (13) comprenant :
une pluralité d'éléments allongés (11) ayant un axe longitudinal, chacun desdits éléments (11) comprenant un adaptateur électroconducteur pouvant être fixé de manière amovible à, et en contact électrique avec, un stent métallique (13) ;
une solution électrolytique (19) comprenant du polyéthylène glycol, de l'éthanol, et du diméthylsulfate contenue dans un récipient de polissage (14) ;
une cathode spiralée continue (21) conçue pour être située à proximité étroite de chacun desdits éléments allongés (11) lorsque lesdits éléments allongés (11) et ladite cathode (21) sont immergés dans ladite solution électrolytique (19) ;
un élément conducteur de courant de cathode fixé à ladite cathode (21) ;
un élément conducteur de courant d'anode dans lequel chacun desdits éléments allongés (11) est raccordé de façon électroconductrice au dit élément conducteur de courant d'anode.

2. Appareil (100) selon la revendication 1, dans lequel le récipient de polissage (14) est fabriqué en un matériau ayant une faible conductivité thermique.

3. Appareil (100) selon l'une quelconque des revendications 1 ou 2, comprenant en outre une seconde cathode spiralée continue (21) conçue pour être située à proximité étroite de chacun desdits éléments allongés (11) lorsque lesdits éléments allongés (11) et ladite cathode (21) sont immergés dans ladite solution électrolytique (19) ; et un second élément conducteur de courant de cathode spiralée fixé à ladite seconde cathode.

4. Appareil (100) selon la revendication 3, dans lequel lesdites cathodes spiralées (21) sont de forme tubulaire et disposées de manière sensiblement concentrique dans ladite solution (19).

5. Procédé de polissage électrolytique simultané de stents métalliques (13) comprenant :
a) la fixation d'un stent (13) sur chacun d'un ou plusieurs adaptateurs électroconducteurs d'un appareil (100), ledit appareil (100) comprenant :
au moins un élément allongé (11) ayant un axe longitudinal, chacun dudit ou desdits éléments (11) comprenant un adaptateur électroconducteur pouvant être fixé de manière amovible à, et en contact électrique avec, un stent métallique (13) ;
une solution électrolytique (19) comprenant du polyéthylène glycol, de l'éthanol, et du diméthylsulfate ;
une cathode spiralée continue (21) conçue pour être située à proximité étroite de chacun dudit ou desdits éléments allongés (11) lorsque ledit ou lesdits éléments allongés (11) et ladite cathode (21) sont immergés dans ladite solution électrolytique (19) ;
un élément conducteur de courant de cathode fixé à ladite cathode (21) ;
un élément conducteur de courant d'anode dans lequel chacun desdits éléments allongés (11) est raccordé de façon électroconductrice au dit élément conducteur de courant d'anode ;
b) l'immersion dudit ou desdits stents (13) dans ladite solution électrolytique (19) ;
c) la fourniture d'une différence de tension entre ledit élément conducteur de courant de cathode et ledit élément conducteur de courant d'anode ;
d) le retrait dudit ou desdits stents (13) de ladite solution (19) et le rinçage avec de l'alcool ; et
e) facultativement, la répétition des points b), c), et d).

6. Procédé selon la revendication 5, comprenant en outre les étapes de :
f) retrait dudit ou desdits stents (13) dudit appareil (100),
g) rinçage dudit ou desdits stents (13) ;
h) immersion dudit ou desdits stents (13) dans une solution de passivation ;
i) retrait dudit ou desdits stents (13) de ladite solution de passivation et rinçage dudit ou desdits stents (13) ; et
j) placement dudit ou desdits stents (13) dans un liquide et application d'une énergie ultrasonore au dit liquide.

7. Procédé selon la revendication 5, dans lequel lesdits points b), c), et d) sont répétés trois à cinq fois.

8. Procédé selon la revendication 6, dans lequel ladite solution de passivation comprend de l'acide nitrique.

9. Procédé selon la revendication 5, dans lequel ladite énergie ultrasonore est appliquée au dit liquide à température ambiante.

10. Solution électrolytique (19) comprenant du polyéthylène glycol, de l'éthanol et du diméthylsulfate.

11. Solution électrolytique (19) selon la revendication 10, dans laquelle le polyéthylène glycol est le PEG 1000.

12. Solution électrolytique (19) selon les revendications 10 ou 11, dans laquelle la solution électrolytique (19) comprend de 0,1 à 5 % en poids de polyéthylène glycol.

13. Solution électrolytique (19) selon l'une quelconque des revendications 10, 11, ou 12, dans laquelle la solution électrolytique (19) comprend de 40 à 70 % en poids de diméthylsulfate, et le reste d'éthanol.
